(19) Europäisches Patentamt
European Patent Office
Office européen des brevets

(11) Veröffentlichungsnummer: **0 035 469**
**A1**

# (12) EUROPÄISCHE PATENTANMELDUNG

(21) Anmeldenummer: **81810055.4**

(22) Anmeldetag: **23.02.81**

(51) Int. Cl.[3]: **C 07 C 149/30**, C 07 C 149/32, A 01 N 31/08

(30) Priorität: **28.02.80 CH 1593/80**
**09.09.80 CH 6763/80**

(43) Veröffentlichungstag der Anmeldung: **09.09.81**
**Patentblatt 81/36**

(84) Benannte Vertragsstaaten: **BE CH DE FR GB IT LI NL**

(71) Anmelder: **CIBA-GEIGY AG, Patentabteilung Postfach, CH-4002 Basel (CH)**

(72) Erfinder: **Brechbühler, Hans U., Dr., Paracelsusstrasse 63, CH-4058 Basel (CH)**
Erfinder: **Ehrenfreund, Josef, Dr., Langenhagweg 11, CH-4123 Allschwil (CH)**

(54) **Phenyl-Alkylthioäther.**

(57) Neue Phenyl-Alkylthioäther der Formel

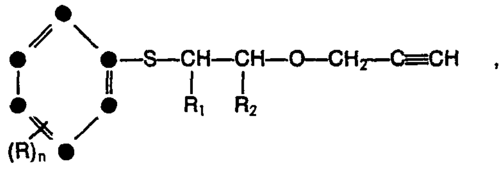

worin R Wasserstoff, Fluor, Chlor, Brom, $C_1$-$C_4$-Alkyl, $C_1$-$C_3$-Alkoxy, $C_3$-Alkenyloxy, mit 1 oder 2 Chloratomen substituiertes $C_3$-Alkenyloxy, $C_3$-Alkinyloxy, Trifluormethyl oder Nitro; $R_1$ und $R_2$ unabhängig voneinander Wasserstoff, Methyl oder Äthyl; und n eine der Zahlen 1 oder 2 bedeuten; Verfahren zur Herstellung dieser Verbindungen sowie diese enthaltende Mittel zur Verwendung in der Schädlingsbekämpfung, insbesondere zur Bekämpfung von Pflanzen und Tiere befallenden Insekten. Die neuen Verbindungen besitzen ausgeprägte ovizid-insektizide Wirkung.

EP 0 035 469 A1

ACTORUM AG

0035469

CIBA-GEIGY AG
Basel (Schweiz)

5-12724/1+2/+

Phenyl-Alkylthioäther

Die vorliegende Erfindung betrifft neue 1-Phenylthio-2-propargyloxyäthane, Verfahren zu ihrer Herstellung und ihre Verwendung in der Schädlingsbekämpfung.

Die erfindungsgemässen 1-Phenylthio-2-propargyloxy-äthane haben die Formel I

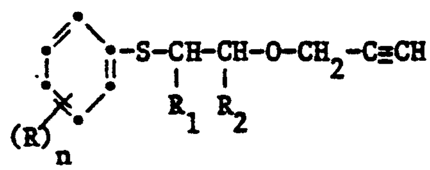

(I),

worin R Wasserstoff, Fluor, Chlor, Brom, $C_1$-$C_4$-Alkyl, $C_1$-$C_3$-Alkoxy, $C_3$-Alkenyloxy, mit 1 oder 2 Chloratomen substituiertes $C_3$-Alkenyloxy, $C_3$-Alkinyloxy, Trifluormethyl oder Nitro;
$R_1$ und $R_2$ unabhängig voneinander Wasserstoff, Methyl oder Aethyl; und n eine der Zahlen 1 oder 2 bedeuten.

Wegen ihrer Wirkung hervorzuheben sind diejenigen Verbindungen der Formel I, worin R Wasserstoff, $C_1$-$C_4$-Alkyl, Fluor, Chlor, Brom, Methoxy, Allyloxy, Propargyloxy oder Trifluormethyl; $R_1$ und $R_2$ unabhängig voneinander Wasserstoff oder Methyl bedeuten.

Eine bevorzugte Gruppe von Verbindungen der Formel I ist ferner dadurch gekennzeichnet, dass $R_1$ und $R_2$ Wasserstoff bedeuten. Ausserdem zeichnen sich diejenigen Verbindungen der Formel I durch besonders gute pestizide Wirksamkeit aus, worin R Methyl oder Chlor und $R_1$ und $R_2$ Wasserstoff bedeuten.

Die Verbindungen der Formel I können nach an sich bekannten Verätherungs-Verfahren unter den hierfür üblichen Bedingungen hergestellt werden [vgl. u.a. "Methoden d. organischen Chemie", Houben-Weyl, Bd. VI/3, S. 11 ff, 116-117 (1965); C.A. Buchler, D.E. Pearson, "Survey of Organic Synthesis", Ch. 6, p. 285 ff (1970); W.P. Weber, G.W. Gokel, "Phase Transfer Catalysis in Organic Synthesis", Ch. 5, p. 77 (1977); S. Patai, "The Chemistry of the Ether Linkage", Ch. 10, p. 445-498 (1967)].

So kann man z.B. die Verbindungen der Formel I mit Hilfe der bekannten Aether-Synthese nach Williamson herstellen:

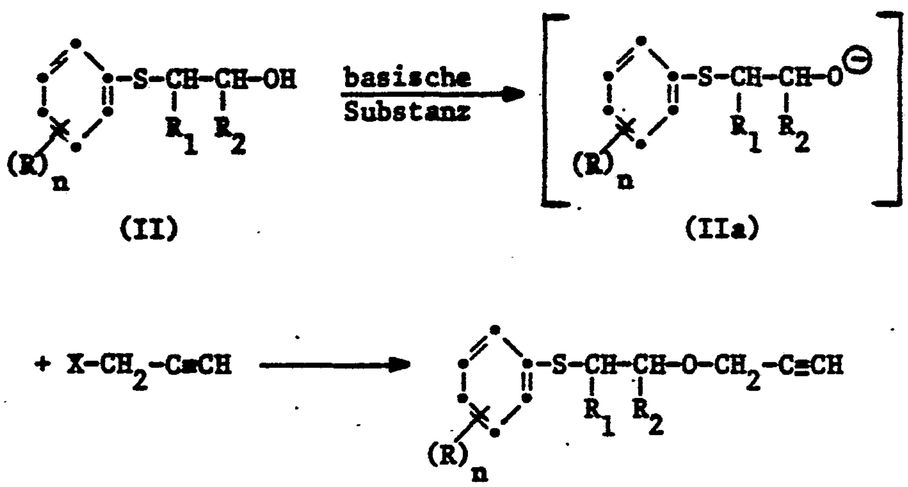


(II) (IIa)

(III) (I)

Bei dieser Synthese werden zur Bildung des Alkoholats der Formel IIa als Kondensationsmittel basische Substanzen, wie Alkalimetallalkoholate niederer Alkohole, Natriumhydrid, Natriumamid, Alkalimetalle, Alkali- oder Erdalkali-Hydroxyde oder -Carbonate, z.B. NaOH, KOH, $Na_2CO_3$, verwendet. Gegebenenfalls kann als Katalysator ein Alkalimetalljodid, z.B. Kaliumjodid, eingesetzt werden. Die Umsetzung wird im allgemeinen unter Normaldruck und bei einer Temperatur von 20-150°C, vorzugsweise 40-120°C, in Gegenwart von aprotischen Lösungsmitteln, z.B. Kohlenwasserstoffen, wie Benzol oder Toluol, höhersiedenden Aethern, wie Dioxan oder Tetrahydrofuran, oder reaktionsfördernden Lösungsmitteln, wie Acetonitril, Dimethylsulfoxid, Hexamethylphosphorsäuretriamid usw., durchgeführt.

Die Verbindungen der Formel I lassen sich auch mittels der sogenannten "Phasentransfer-Katalyse" herstellen, und zwar durch Umsetzung einer Verbindung der Formel II mit aktivierten Propargylverbindungen der Formel III, z.B. Propargylbromid. Man kann mit Hilfe der Phasentransfer-Katalyse zu den Verbindungen der Formel I auch dadurch gelangen, dass man eine Verbindung der Formel IV

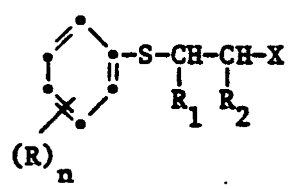

(IV)

mit Propargylalkohol umsetzt.

Diese Umsetzungen werden im allgemeinen unter Normaldruck und bei einer Reaktionstemperatur, die zwischen Raumtemperatur und 80°C, vorzugsweise zwischen 20 und 60°C, liegt, durchgeführt, wobei ein Alkalimetallhydroxyd, z.B. Natriumhydroxyd, als Kondensationsmittel dient. Als Katalysatoren kommen die bekannten Phasentransfer-Katalysatoren, vorzugsweise Tetrabutylammoniumbisulfat, in Betracht. Die Lösungsmittel-Kombination für die Phasentransfer-Reaktion bestehen aus einer wässrigen und einer mit Wasser nicht mischbaren Phase, z.B. 50%-iger wässrigen Natriumhydroxyd-Lösung und Methylenchlorid.

Ferner sind die Verbindungen der Formel I erhältlich durch Umsetzung eines Alkohols der Formel II mit Propargylalkohol in Gegenwart eines Kondensationsmittels. Als Kondensationsmittel kann eine wasserbindende Substanz, z.B. ein Carbodiimid, wie Dicyclohexylcarbodiimid (vgl. Chem. Rev. 53, 145/1953), oder eine Säure, z.B. konzentrierte Salzsäure (vgl. Chem. Abstracts 79, 66110/1973), verwendet werden. Diese Umsetzungen können mit oder ohne Lösungsmittel, gegebenenfalls bei erhöhter Temperatur, durchgeführt werden.

In den vorstehenden Formeln I, II, IIa, III und IV haben die Reste R, $R_1$ und $R_2$ sowie n die unter Formel I angegebenen Bedeutungen, X bedeutet eine geeignete Abgangsgruppe, wie z.B. Chlor, Brom, Jod oder Sulfonat, wie Mesylat oder Tosylat.

Die als Ausgangsstoffe verwendeten Verbindungen der Formeln II, III und IV sind bekannt oder können analog bekannten Verfahren erhalten werden. Beispielsweise können die gegebenenfalls substituierten Phenylthioäthanol-Derivate der Formel II gemäss den allgemein üblichen Arbeitsweisen für die Kondensation von Thiophenolen mit aliphatischen Halogeniden wie folgt hergestellt werden:

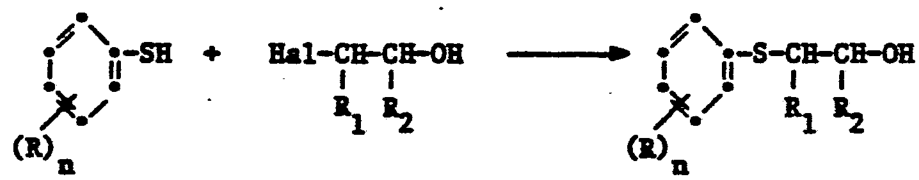

(II)

In den obigen Formeln haben R, $R_1$, $R_2$ und n die unter Formel I angegebenen Bedeutungen und Hal steht für Halogen, vorzugsweise für Chlor oder Brom.

Aus der deutschen Offenlegungsschrift Nr. 2 803 806 sind bereits α-Phenoxy-ω-halogenalkinyloxy-alkane und aus der DDR-Patentschrift Nr. 103 543 substituierte 1-Phenoxy-2-alkoxy-äthane als Insektizide mit Juvenilhormon-Wirksamkeit bekannt. Ferner werden in der deutschen Offenlegungsschrift Nr. 2 518 018 1-(4-Phenylthio)-, 1-(4-Benzoyl)- und 1-(4-Benzyl)-phenylthio-2-alkinyloxy-äthane als Insektizide beschrieben. Substituierte Phenylthio-alkyloxy-äthanderivate werden in Chem. Abstr. 69, 34969 als Synergisten für Insektizide und in der Japanischen Patentschrift Nr. 7 335 451 als Herbizide und Akarizide vorgeschlagen.

Ueberraschenderweise wurde nun gefunden, dass die erfindungsgemässen 1-Phenylthio-2-propargyloxy-äthane der Formel I bei guter Pflanzenverträglichkeit und geringer Warmblütertoxizität ausgezeichnete Wirksamkeit als Schädlingsbekämpfungsmittel, vor allem als ovizid und ovolarvizid wirkende Insektizide, zur Bekämpfung von Pflanzen und Tiere befallenden Schädlingen aufweisen.

Insbesondere eignen sich die Verbindungen der Formel I zur Bekämpfung von Insekten der Ordnungen: Lepidoptera, Coleoptera, Homoptera, Heteroptera, Diptera, Thysanoptera, Orthoptera, Anoplura, Siphonaptera, Mallophaga, Thysanura, Isoptera, Psocoptera und Hymenoptera.

Neben ihrer sehr günstige Wirkung gegenüber Fliegen, wie z.B. Musca domestica, und Mückenlarven eignen sich Verbindungen der Formel I auch zur Bekämpfung von pflanzenschädigenden Frassinsekten, in Zier- und Nutzpflanzungen, insbesondere in Baumwollkulturen (z.B. Spodoptera littoralis und Heliothis virescens) sowie in Obst- und Gemüsekulturen (z.B. Laspeyresia pomonella und Leptinotarsa decemlineata). Hervorzuheben ist besonders die hohe ovizide Wirkung der Verbindungen der Formel I.

Die Wirkung der erfindungsgemässen Verbindungen bzw. der sie enthaltenden Mittel lässt sich durch Zusatz von anderen Insektiziden und/oder Akariziden wesentlich verbreitern und an gegebene Umstände anpassen.

Als Zusätze kommen z.B. folgende Wirkstoffe in Betracht:
organische Phosphorverbindungen,
Nitrophenole und Derivate,
Formamidine, Harnstoffe,
Carbamate, Pyrethroide und
chlorierte Kohlenwasserstoffe.

Die Verbindungen der Formel I können für sich allein oder zusammen mit geeigneten Trägern und/oder Zuschlagstoffen eingesetzt werden. Geeignete Träger und Zuschlagstoffen können fest oder flüssig sein und entsprechen den in der Formulierungstechnik üblichen Stoffen, wie z.B. natürlichen oder regenerierten Stoffen, Lösungs-, Dispergier-, Netz-, Haft-, Verdickungs-, Bind- und/oder Düngemitteln. Zur Applikation können die Verbindungen der Formel zu Stäubemitteln, Emulsionskonzentraten, Granulaten, Dispersionen, Sprays, zu Lösungen oder Aufschlämmungen in üblicher Formulierung, die in der Applikationstechnik zum Allgmeinwissen gehören, verarbeitet werden.

Die Herstellung erfindungsgemässer Mittel erfolgt in an sich bekannter Weise durch inniges Vermischen und/oder Vermahlen von Wirkstoffen der Formel I mit geeigneten Trägerstoffen, gegebenenfalls unter Zusatz von gegenüber den Wirkstoffen inerten Dispergier- und Lösungsmitteln. Die Wirkstoffe können in den folgenden Aufarbeitungsformen vorliegen und angewendet werden:

Feste Aufarbeitungsformen: Stäubemittel, Streumittel, Granulate (Umhüllungsgranulate, Imprägnierungsgranulate und Homogengranulate);

Flüssige Aufarbeitungsformen:

a) in Wasser dispergierbare Wirkstoffkonzentrate: Spritzpulver (wettable powders), Pasten, Emulsionen;

b) Lösungen.

Der Gehalt an Wirkstoff in den oben beschriebenen Mitteln liegt im allgemeinen zwischen 0,1 bis 95%.

Die Wirkstoffe der Formel I können beispielsweise wie folgt formuliert werden:

Stäubemittel: Zur Herstellung eines a) 5%igen und b) 2%igen Stäubemittels werden die folgenden Stoffe verwendet:

a) 5 Teile Wirkstoff,
95 Teile Talkum;

b) 2 Teile Wirkstoff,
1 Teil hochdisperse Kieselsäure,
97 Teile Talkum.

Die Wirkstoffe werden mit den Trägerstoffen vermischt und vermahlen.

Granulat: Zur Herstellung eines 5%igen Granulates werden die folgenden Stoffe verwendet:

5,00 Teile Wirkstoff,
0,25 Teile epoxydiertes Pflanzenöl,
0,25 Teile Cetylpolyglykoläther,
3,50 Teile Polyäthylenglykol,
91,00 Teile Kaolin (Korngrösse 0,3-0,8 mm).

Die Aktivsubstanz wird mit dem epoxydiertes Pflanzenöl vermischt und mit 6 Teilen Aceton gelöst, hierauf wird Polyäthylenglykol und Cetylpolyglykoläther zugesetzt. Die so erhaltene Lösung wird auf Kaolin aufgesprüht und anschliessend das Aceton im Vakuum verdampft.

Spritzpulver: Zur Herstellung eines a) 40%igen, b) und c) 25%igen, d) 10%igen Spritzpulvers werden folgende Bestandteile verwendet:

a) 40 Teile Wirkstoff,
5 Teile Ligninsulfonsäure-Natriumsalz,
1 Teile Dibutylnaphthalinsulfonsäure-Natriumsalz,
54 Teile Kieselsäure;

0035469

b) 25,0 Teile Wirkstoff,
4,5 Teile Calcium-Ligninsulfonat,
1,9 Teile Champagne-Kreide/Hydroxyäthylcellulose-Gemisch (1:1),
1,5 Teile Natrium-dibutyl-naphthalinsulfonat,
19,5 Teile Kieselsäure,
19,5 Teile Champagne-Kreide,
28,1 Teile Kaolin;

c) 25,0 Teile Wirkstoff,
2,5 Teile Isooctylphenoxy-polyoxyäthylen-äthanol,
1,7 Teile Champagne-Kreide/Hydroxyäthylcellulose-Gemisch (1:1),
8,3 Teile Natriumaluminiumsilikat,
16,5 Teile Kieselgur,
46,0 Teile Kaolin;

d) 10 Teile Wirkstoff,
3 Teile Gemisch der Natriumsalze von gesättigten Fettalkoholsulfaten,
5 Teile Naphthalinsulfonsäure/Formaldehyd-Kondensat,
82 Teile Kaolin.

Die Wirkstoffe werden in geeigneten Mischern mit den Zuschlagstoffen innig vermischt und auf entsprechenden Mühlen und Walzen vermahlen. Man erhält Spritzpulver, die sich mit Wasser zu Suspensionen jeder gewünschten Konzentration verdünnen lassen.

Emulgierbare Konzentrate: Zur Herstellung eines a) 10%igen, b) 25%igen und c) 50%igen emulgierbaren Konzentrates werden folgende Stoffe verwendet:

a) 10,0 Teile Wirkstoff,
3,4 Teile epoxydiertes Pflanzenöl,
3,4 Teile eines Kombinationsemulgators, bestehend aus Fettalkoholpolyglykoläther und Alkylaralkylsulfonat-Calcium-Salz,

40,0 Teile Dimethylformamid,
43,2 Teile Xylol;

b) 25,0 Teile Wirkstoff,
2,5 Teile epoxydiertes Pflanzenöl,
10,0 Teile eines Alkylarylsulfonat/Fettalkoholpolyglykoläther-Gemisches,
5,0 Teile Dimethylformamid,
57,5 Teile Xylol;

c) 50,0 Teile Wirkstoff,
4,2 Teile Tributylphenol-Polyglykoläther,
5,8 Teile Calcium-Dodecylbenzolsulfonat,
20,0 Teile Cyclohexanon,
20,0 Teile Xylol.

Aus solche Konzentrationen können durch Verdünnen mit Wasser Emulsionen jeder gewünschten Konzentration hergestellt werden.

Sprühmittel: Zur Herstellung eines a) 5%igen und b) 95%igen Sprühmittels werden die folgenden Bestandteile verwendet:

a) 5 Teile Wirkstoff,
1 Teil epoxydiertes Pflanzenöl,
94 Teile Benzin (Siedegrenzen 160-190°C);

b) 95 Teile Wirkstoff,
5 Teile epoxydiertes Pflanzenöl.

Beispiel 1: 7,7 g (0,05 Mol) 2-Phenylthioäthanol, 6,6 g Propargylbromid, 25 ml 50%ige Natronlauge und 50 ml Methylenchlorid werden unter Zusatz von 0,5 g Tetrabutylammoniumhydrogensulfat während 20 Stunden auf 40°C gehalten. Nach dem Abkühlen werden die Phasen getrennt. Die organische Phase wird mit Wasser gewaschen und mittels $Na_2SO_4$

getrocknet. Man erhält ein gelbes Oel, das nach Chromatographie am Kieselgel (Laufmittel: Hexan/Aether, 5:1) 1-Phenylthio-2-propargyloxyäthan ($n_D^{20}$ = 1,5625) ergibt.

Analog den vorstehend beschriebenen Arbeitsweisen werden auch die folgenden in Tabelle I aufgeführten Verbindungen der Formel I hergestellt:

Tabelle I

| Verbindung Nr. | R | $R_1$ | $R_2$ | n | physikalische Daten |
|---|---|---|---|---|---|
| 1 | 4-Cl | $CH_3$ | $CH_3$ | 1 | $n_D^{20}$ = 1,5625 |
| 2 | 4-Cl | H | H | 1 | $n_D^{20}$ = 1,5709 |
| 3 | H | H | H | 1 | $n_D^{20}$ = 1,5625 |
| 4 | 4-tert.$C_4H_9$ | H | H | 1 | $n_D^{20}$ = 1,5425 |
| 5 | 4-$CH_3$ | H | H | 1 | $n_D^{20}$ = 1,5560 |
| 6 | 4-Br | H | H | 1 | $n_D^{20}$ = 1,5935 |
| 7 | 4-$OCH_3$ | H | H | 1 | $n_D^{20}$ = 1,5610 |
| 8 | 4-O-$CH_2$-C≡CH | H | H | 1 | $n_D^{20}$ = 1,5695 |
| 9 | 3-$CF_3$ | H | H | 1 | $n_D^{20}$ = 1,5050 |
| 10 | 4-F | H | H | 1 | $n_D^{20}$ = 1,5400 |
| 11 | 3-$OCH_3$ | H | H | 1 | $n_D^{20}$ = 1,5640 |
| 12 | 2-Cl, 5-Cl | H | H | 2 | $n_D^{20}$ = 1,5890 |

| Verbindung Nr. | R | $R_1$ | $R_2$ | n | physikalische Daten |
|---|---|---|---|---|---|
| 13 | 3-Cl, 4-Cl | H | H | 2 | $n_D^{20}$= 1,5850 |
| 14 | 3-$CH_3$ | H | H | 1 | $n_D^{20}$= 1,5575 |
| 15 | 4-O-$CH_2$-CH=$CH_2$ | H | H | 1 | $n_D^{20}$= 1,5597 |
| 16 | H | $CH_3$ | H | 1 | $n_D^{20}$= 1,5495 |
| 17 | H | $C_2H_5$ | H | 1 | $n_D^{20}$= 1,5470 |
| 18 | H | H | $CH_3$ | 1 | |
| 19 | H | H | $C_2H_5$ | 1 | |
| 20 | 4-Cl | $CH_3$ | H | 1 | $n_D^{20}$= 1,5615 |
| 21 | 4-Cl | $C_2H_5$ | H | 1 | $n_D^{20}$= 1,5565 |
| 22 | 4-Cl | H | $CH_3$ | 1 | |
| 23 | 4-Cl | H | $C_2H_5$ | 1 | |
| 24 | 4-O-$CH_2$-CH=$CH_2$ | $CH_3$ | H | 1 | |
| 25 | 4-O-$CH_2$-CH=$CH_2$ | $C_2H_5$ | H | 1 | |
| 26 | 4-O-$CH_2$CH=$CH_2$ | H | $CH_3$ | 1 | |
| 27 | 4-O-$CH_2$-CH=$CH_2$ | H | $C_2H_5$ | 1 | |
| 28 | 4-$OC_2H_5$ | $CH_3$ | $CH_3$ | 1 | |
| 29 | 3-Br | H | H | 1 | $n_D^{20}$= 1,5902 |
| 30 | 2-Cl, 3-Cl | H | H | 2 | $n_D^{20}$= 1,5836 |
| 31 | 3-Cl, 5-Cl | H | H | 2 | $n_D^{20}$= 1,5831 |
| 32 | 2-Cl, 4-Cl | H | H | 2 | $n_D^{20}$= 1,5871 |

| Verbindung Nr. | R | $R_1$ | $R_2$ | n | physikalische Daten |
|---|---|---|---|---|---|
| 33 | $4-OC_2H_5$ | H | H | 1 | |
| 34 | $4-O-CH(CH_3)_2$ | $CH_3$ | H | 1 | |
| 35 | $4-O-CH(CH_3)_2$ | H | H | 1 | $n_D^{20} = 1,5420$ |
| 36 | $4-NO_2$ | H | H | 1 | |
| 37 | $4-O-CH_2-CH=CHCl$ | H | H | 1 | $n_D^{20} = 1,5700$ |
| 38 | $4-C_2H_5$ | H | H | 1 | $n_D^{20} = 1,5514$ |

Beispiel 2: Kontaktwirkung auf Musca domestica:

In jeweils eine Petrischale (9 cm Durchmesser) mit einer geätzten Innenfläche wurde 1 ml einer acetonischen Lösung enthaltend 1 bzw. 5 mg des zu prüfenden Wirkstoffes pipettiert. Die Wirkstofflösung wurde gleichmässig auf dem Schalenboden verteilt, wobei das Aceton verdampfte. Eine Stunde nach Verdampfen des Acetons wurden pro behandelte Schale 10 drei Tage alte, in Kälterstarre befindliche, normalsensible adulte Fliegen (Musca domestica) angesetzt. Die Ansätze wurden bei Raumtemperatur gehalten.

Die Auswertung erfolgte aufgrund des Prozentsatzes der durch den Kontakt mit der behandelten Unterlage abgetöteten Tiere (% Rückenlage).

Verbindungen gemäss Beispiel 1 zeigten gute Wirkung in obigem Test.

Beispiel 3: Kontaktwirkung auf Aedes aegypti (Larven):

Mittels einer Pipette wurde 1 ml einer acetonischen Lösung der zu prüfenden Wirksubstanz auf die Oberfläche von 100 ml in einem Plastikbecher befindlichem Wasser gegeben. Die acetonische Lösung enthielt die Wirksubstanz in einer solchen Menge, dass sich Wirkstoffkonzentrationen von 10 bzw. 1 ppm in dem im jeweiligen Becher enthaltenen Wasser ergaben. Nach Verdampfen des Acetons und einer Wartezeit von einer Stunde wurden etwa 50 viertägige Aedes-Larven in jeden der den Wirkstoff enthaltenden Becher eingesetzt. Die Ansätze wurden bei Raumtemperatur gehalten.

Zur Auswertung wurde nach 2, 4 und 24 Stunden sowie nach 8 Tagen auf Mortalität, d.h. den Prozentsatz der nicht mehr schwimmfähigen Larven, geprüft.

Verbindungen gemäss Beispiel 1 zeigten gute Wirkung im obigen Test.

Beispiel 4: Wirkung auf Spodoptera littoralis (ovizid):

Es wurde eine benetzbare, pulverförmige Formulierung oder ein emulgierbares Konzentrat enthaltend 25 Gew.-% des zu prüfenden Wirkstoffes hergestellt. Hieraus wurden durch Zugabe von Wasser benetzungsfähige Emulsions-Zubereitungen mit Wirkstoffkonzentrationen von 400 ppm und 800 ppm erhalten. Auf Filterpapier abgelegt, zwei Tage alte Eier von Spodoptera wurden in die wässrige Wirkstoff-Zubereitung eingetaucht, herausgenommen und in Plastikschalen bei 28°C und 60% relativer Feuchtigkeit gehalten.

Die Auswertung erfolgte nach 14 Tagen durch Bestimmung der relativen Zahl der aus den behandelten Eiern ausgeschlüpften Larven (prozentuale Schlupfrate).

Die Wirkung von Verbindungen gemäss Beispiel 1 im obigen Test geht aus der nachstehenden Tabelle II hervor.

**Beispiel 5: Ovizide Wirkung auf Epilachna varivestis:**

Es wurden 20 Gew.-% Wirkstoff, 70 Gew.-% Xylol und 10 Gew.-% einer Mischung aus einem Reaktionsprodukt eines Alkylphenoles mit Aethylenoxid und Calcium-dodecylbenzolsulfonat miteinander vermischt. Aus diesem Konzentrat wurden wässrige Emulsionen enthaltend 800 und 1600 ppm Wirkstoff hergestellt.

Jeweils ca. 100 auf Blätter von Phaseolus vulgaris frisch abgelegte Eier von Epilachna varivestis (mexikanischer Bohnenkäfer) wurden mit den oben beschriebenen wässrigen Emulsionen (Konzentration 800 bzw. 1600 ppm Wirkstoff) angefeuchtet und leicht getrocknet.

In einem gelüfteten Gefäss wurden die behandelten Gelege solange gehalten, bis die gleichzeitig angesetzten unbehandelten Kontrollen geschlüpft waren. Unter einem Binocular erfolgte Auswertung hinsichtlich der erzielten prozentualen Abtötung.

Verbindungen gemäss Beispiel 1 zeigten gute Wirkung in obigem Test.

**Beispiel 6: Ovizide Wirkung auf Heliothis virescens und Leptinotarsa decemlineata:**

Entsprechende Mengenanteile einer benetzbaren pulverförmigen Formulierung oder eines emulgierbaren Konzentrates, enthaltend 25 Gew.-% des zu prüfenden Wirkstoffes, wurden mit jeweils soviel Wasser vermischt, dass sich wässrige Emulsionen von ansteigender Wirkstoffkonzentration ergaben.

In diese wirkstoffhaltigen Emulsionen wurden eintägige Eilege von Heliothis auf Cellophan bzw. Eigelege von Leptinotarsa auf Kartoffelblättern während drei Minuten eingetaucht und dann auf Rundfiltern abgenutscht. Die so behandelten Gelege wurden in Petrischalen ausgelegt und in der Dunkelheit aufbewahrt. Nach 6 bis 8 Tagen wurde die Schlupfrate im Vergleich zu unbehandelten Kontrollen festgestellt. Zur Auswertung wurde die zur 100%igen Abtötung der Eier erforderliche minimale Wirkstoffkonzentration bestimmt.

Die Wirkung von Verbindungen gemäss Beispiel 1 in diesem Test geht aus der nachstehenden Tabelle II hervor.

Beispiel 7: Wirkung von Laspeyresia pomonella (Eier):

Abgelegte Eier von Laspeyresia pomonella, die nicht älter als 24 Stunden waren, wurden auf Filterpapier für 1 Minute in eine acetonisch-wässrige Lösung enthaltend 200 ppm und 400 ppm des zu prüfenden Wirkstoffes eingetaucht. Nach dem Antrocknen der Lösung wurden die Eier in Petrischalen ausgelegt und bei einer Temperatur von 28°C belassen. Nach 6 Tagen wurde der prozentuale Schlupf aus den behandelten Eiern bewertet.

Die Wirkung von Verbindungen gemäss Beispiel 1 in diesem Test geht aus der Tabelle II hervor.

Biologische Versuchsergebnisse

In Tabelle II sind Versuchsergebnisse auf der Basis der vorstehenden Beispiele aufgeführt, und zwar mit folgendem Bewertungsindex in Bezug auf die prozentuale Abtötung der Schädlinge:

A: 70-100% Abtötung bei 50 ppm Wirkstoffkonzentration
B: 70-100% Abtötung bei 100 ppm Wirkstoffkonzentration
C: 70-100% Abtötung bei 200 ppm Wirkstoffkonzentration
D: 70-100% Abtötung bei 400 ppm Wirkstoffkonzentration

X: 0- 70% Abtötung bei 800 ppm Wirkstoffkonzentration
-: nicht geprüft

Tabelle II

| Verbindung | Wirksamkeit | | |
|---|---|---|---|
| Nr. | Spodoptera ovizid (Beispiel 4) | Heliothis ovizid (Beispiel 6) | Laspeyresia ovizid (Beispiel 7) |
| 1 | D | D | - |
| 2 | D | A | C |
| 3 | D | D | D |
| 4 | D | A | - |
| 5 | D | A | D |
| 6 | D | A | - |
| 7 | D | - | D |
| 8 | D | A | - |
| 9 | D | A | - |
| 10 | D | B | - |
| 11 | D | A | C |
| 12 | D | A | - |
| 13 | D | A | C |
| 14 | D | B | C |

Tabelle II - Fortsetzung

| Verbindung Nr. | Wirksamkeit Spodoptera ovizid (Beispiel 4) | Heliothis ovizid (Beispiel 6) | Laspeyresia ovizid (Beispiel 7) |
|---|---|---|---|
| 15 | D | B | - |
| 16 | D | X | - |
| 17 | D | X | - |
| 20 | D | C | - |
| 21 | D | X | - |
| 29 | D | A | - |
| 30 | D | A | - |
| 31 | D | B | - |
| 32 | D | A | - |
| 35 | D | B | C |
| 37 | D | B | - |
| 38 | D | A | - |

Patentansprüche

1. Verbindung der Formel I

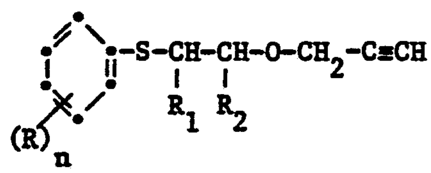

(I) ,

worin R Wasserstoff, Fluor, Chlor, Brom, $C_1$-$C_4$-Alkyl, $C_1$-$C_3$-Alkoxy, $C_3$-Alkenyloxy, mit 1 oder 2 Chloratomen substituiertes $C_3$-Alkenyloxy, $C_3$-Alkinyloxy, Trifluormethyl oder Nitro;
$R_1$ und $R_2$ unabhängig voneinander Wasserstoff, Methyl oder Aethyl; und
n eine der Zahlen 1 oder 2 bedeuten.

2. Verbindung der Formel I gemäss Anspruch 1, dadurch gekennzeichnet, dass R Wasserstoff, $C_1$-$C_4$-Alkyl, Fluor, Chlor, Brom, Methoxy, Allyloxy, Propargyloxy oder Trifluormethyl; und $R_1$ und $R_2$ unabhängig voneinander Wasserstoff oder Methyl bedeuten.

3. Verbindung der Formel I gemäss Anspruch 2, dadurch gekennzeichnet, dass $R_1$ und $R_2$ Wasserstoff bedeuten.

4. Verbindung der Formel I gemäss Anspruch 2, dadurch gekennzeichnet, dass R Methyl oder Chlor und $R_1$ und $R_2$ Wasserstoff bedeuten.

5. Verbindung gemäss Anspruch 4 der Formel

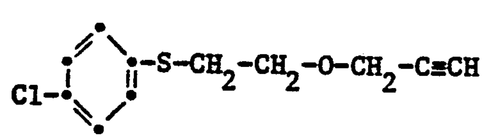

.

6. Verbindung gemäss Anspruch 4 der Formel

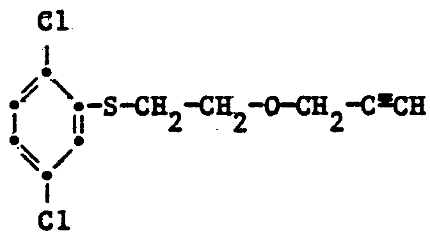 .

7. Verbindung gemäss Anspruch 4 der Formel

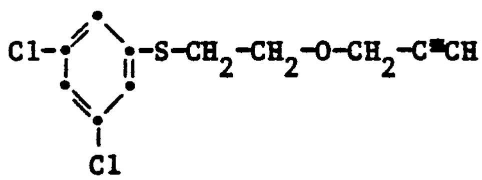 .

8. Verbindung gemäss Anspruch 4 der Formel

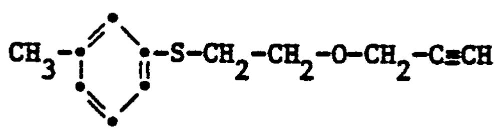 .

9. Verbindung gemäss Anspruch 4 der Formel

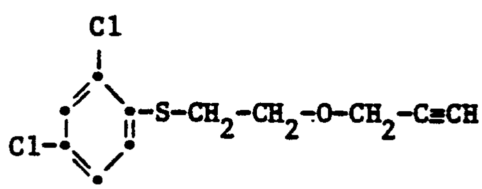 .

10. Verfahren zur Herstellung einer Verbindung gemäss den Ansprüchen 1 bis 9, dadurch gekennzeichnet, dass man in Gegenwart eines Kondensationsmittels

a) eine Verbindung der Formel II

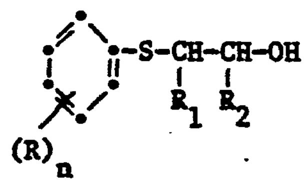

(II)

mit einer Verbindung der Formel III

$CH\equiv C-CH_2-X$ (III)

oder

b) eine Verbindung der Formel IV

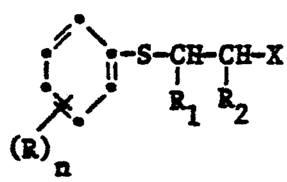

(IV)

mit Propargylalkohol umsetzt, wobei in den Formeln II, III und IV die Reste R, $R_1$ und $R_2$ sowie n die in den Ansprüchen 1 bis 4 angegebenen Bedeutungen haben, X für Chlor, Brom, Jod, Sulfonat, insbesondere Mesylat und Tosylat, oder die OH-Gruppe steht.

11. Schädlingsbekämpfungsmittel, welches als aktive Komponente eine Verbindung gemäss den Ansprüchen 1 bis 9 zusammen mit geeigneten Trägern und/oder Zuschlagstoffen enthält.

12. Verwendung einer Verbindung gemäss den Ansprüchen 1 bis 9 zur Bekämpfung von Schädlingen, vorzugsweise von Insekten.

13. Verwendung einer Verbindung gemäss den Ansprüchen 1 bis 9 als Ovizide zur Bekämpfung pflanzenschädigender Insekten.

Europäisches Patentamt

# EUROPÄISCHER RECHERCHENBERICHT

0035469
Nummer der Anmeldung
EP 81 81 0055

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| D | DE - A - 2 518 018 (CIBA-GEIGY)<br>* Insgesamt *<br>-- | 1 |
| D | CHEMICAL ABSTRACTS, Band 69, Nr. 9, 26. August 1968, Seite 3252, Spalte 1, Nr. 34969n<br>Columbus, Ohio, U.S.A.<br>YA.A. MANDEL'BAUM et al.: "Substituted phenoxy- and phenylthiodiethyl sulfides as synergists for organophosphorus insecticides"<br>& KHIM. PROM. 1968, 44(5), 341-343<br>* Zusammenfassung *<br>---- | 1 |

KLASSIFIKATION DER ANMELDUNG (Int Cl.³)

C 07 C 149/30
149/32
A 01 N 31/08

RECHERCHIERTE SACHGEBIETE (Int. Cl.³)

C 07 C 149/30
149/32

KATEGORIE DER GENANNTEN DOKUMENTE

- X: von besonderer Bedeutung
- A: technologischer Hintergrund
- O: nichtschriftliche Offenbarung
- P: Zwischenliteratur
- T: der Erfindung zugrunde liegende Theorien oder Grundsätze
- E: kollidierende Anmeldung
- D: in der Anmeldung angeführtes Dokument
- L: aus andern Gründen angeführtes Dokument
- &: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 22-05-1981 | GRAMAGLIA |